# FASCICULE DE BREVET EUROPEEN

(11) **EP 0 636 187 B1**
(45) Date de publication et mention de la délivrance du brevet: **30.08.2000**
(21) Numéro de dépôt: 94905767.3
(22) Date de dépôt: 31.01.1994
(51) Int. Cl.: C12Q 1/68, C07H 21/00, C07H 21/04, C12N 15/63

(54) **SEQUENCES NUCLEOTIDIQUES HYBRIDANT SPECIFIQUEMENT AVEC UNE SEQUENCE NUCLEIQUE GENOMIQUE DE CAMPYLOBACTER JEJUNI**
NUKLEOTIDSEQUENZEN DIE SPEZIFISCH MIT EINER NUKLEOTIDSEQUENZ AUS CAMPYLOBACTER JEJUNI HYBRIDISIEREN
NUCLEOTIDIC SEQUENCES HYBRIDIZING SPECIFICALLY WITH A CAMPYLOBACTER JEJUNI GENOMIC NUCLEIC SEQUENCE

(30) Priorité: 29.01.1993 FR 9300978
(43) Date de publication de la demande: 01.02.1995
(73) Titulaire: INSTITUT PASTEUR, 75724 Paris Cédex 15 (FR)
(72) Inventeur: GUESDON, Jean-Luc, F-92310 Sèvres (FR); STONNET, Véronique, F-92600 Asnières (FR)
(74) Mandataire: Le Guen, Gérard
(86) Numéro de dépôt international: FR9400122
(87) Numéro de publication internationale: WO9417205

(56) Documents cités:
- WO-A-86/04422
- J CLIN MICROBIOL 30 (10). 1992. 2613-2619. CODEN: JCMIDW ISSN: 0095-1137 6 - (C) FILE BIOSIS OYOFO B A et al 'SPECIFIC DETECTION OF CAMPYLOBACTER -JEJUNI AND CAMPYLOBACTER -COLI BY USING POLYMERASE CHAIN REACTION.'
- PATENT ABSTRACTS OF JAPAN vol. 15, no. 302 (C-0855) & JP,A,03 112 498 (SHIMADZU CORP) 14 Mai 1991
- NUCLEIC ACIDS RESEARCH vol. 14, no. 15 , 1986 , ARLINGTON, VIRGINIA US pages 6115 - 6128 JABLONSKI ET AL. cité dans la demande
- JOURNAL OF CLINICAL MICROBIOLOGY vol. 27, no. 2 , Février 1989 , WASHINGTON US pages 321 - 326 CHEVRIER ET AL. 'Identification and classification of Campylobacter strains by using nonradioactive DNA probes' cité dans la demande
- ZENTRALBLATT FüR BAKTERIOLOGIE vol. 272 , 1989 , STUTTGART, DE pages 186 - 190 ZHOU AND WANG 'Application of a biotin labelled DNA probe to detect Campylobacter' cité dans la demande

## Description

La présente invention se rapporte à une séquence nucléique spécifique de *Campylobacter jejuni*, ainsi qu'aux applications de cette séquence en tant que sonde nucléotidique spécifique pour la détection de séquences de *Campylobacter jejuni* ou de fragments de cette séquence comme amorces nucléotidiques pour l'amplification d'ADN ou d'ARN de *Campylobacter jejuni* dans un échantillon biologique.

Les infections à *Campylobacter* sont très répandues dans le monde entier, affectant aussi bien l'homme que les animaux sauvages ou domestiques.

Bien que découvertes au début du vingtième siècle, les bactéries appelées actuellement *Campylobacter* furent longtemps méconnues, car leurs particularités rendaient leur identification et leur culture difficiles. D'abord isolés chez les ovidés et les bovidés et appelés *Vibrio fetus* puis, plus tard, *Campylobacter fetus*, ce n'est qu'à partir de 1946 que les premiers cas de campylobactérioses humaines ont été décrits, mais ce n'est qu'à partir de 1972, lorsque des milieux sélectifs pour *Campylobacter* ont commencé à être mis au point, que l'importance des infections à *Campylobacter* a pu être prouvée et reconnue.

Depuis la désignation de l'espèce type *Campylobacter fetus*, une douzaine d'autres espèces et sous-espèces ont été découvertes, le nombre exact variant selon les auteurs et les méthodes taxonomiques, qui proposent souvent de nouveaux critères de classification. Parmi ces espèces, les plus rencontrées en pathologie humaine et/ou animale sont *Campylobacter jejuni*, *Campylobacter coli* et *Campylobacter fetus*.

Actuellement, *Campylobacter jejuni* est considérée comme l'une des causes les plus fréquentes de diarrhée infectieuse chez l'homme.

Le "réseau de surveillance nationale des infections à *Campylobacter*", mis en place en France en 1986, publie chaque année un bilan faisant ressortir les principales données épidémiologiques et cliniques des cas rapportés. Pour les années 1988, 1989 et 1990 par exemple, il apparait que l'espèce la plus impliquée dans ces infections (de 60 à 75% des cas analysés) était *C. jejuni*.

Dans l'espèce humaine, le symptôme majeur de l'infection intestinale à *C. jejuni* est la diarrhée qui, dans les cas les plus graves peut entrainer de fortes pertes hydriques, ce qui peut être particulièrement dangereux pour les enfants et les nourrissons, très sensibles à la déshydratation. Cependant, l'entérite à *C. jejuni* reste souvent sans complications et les diarrhées peuvent même cesser spontanément au bout d'une semaine. Toutefois, les coprocultures peuvent rester positives jusqu'à quelques semaines ou même mois, et, dans 5 à 10% des cas, des rechutes peuvent survenir. Un traitement et un suivi rigoureux s imposent donc, surtout pour les sujets immunodéprimés ou ayant des maladies graves (SIDA, cirrhose du foie, cancer, leucémie, etc.), chez lesquels les *Campylobacter* peuvent se comporter comme des bactéries opportunistes.

D'autres conséquences des infections à *C. jejuni* ont également été décrites, bien que plus rares ou exceptionnelles : adénite mésentérique, cholécystite, infections urinaires, méningites, septicémies, érythème noueux ou syndrome de Guillain-Barré, etc.

Chez les animaux, les *Campylobacter* vivent habituellement en commensaux dans le tube digestif de nombreuses espèces : bovins, ovins, porcs, volailles, oiseaux sauvages, chiens et chats. Ces animaux, malades ou porteurs sains, constituent un large réservoir de germes, et donc un risque important de contamination. Dans le cas d'infections évidentes, chez les bovins et ovins, *C. jejuni* est connu, depuis la première description en 1931, comme étant la cause de la "dysenterie du bétail", qui peut avoir pour conséquence, outre l'effet sur le bétail, la transmission à l'homme à travers la dissémination des germes dans l'environnement des animaux (terre, eaux). Même pour des animaux asymptomatiques, "porteurs sains", la transmission à l'homme peut se faire : soit par contact direct avec ces animaux ou leurs déjections, soit par consommation d'aliments ou d'eaux souillés (viandes contaminées pendant leur préparation et mal cuites, lait non pasteurisé, eau polluée, etc.).

Dans une optique de prévention, il est important donc, tant chez l'homme que chez l'animal, de pouvoir identifier le pathogène *C. jejuni* le plus tôt possible, afin d'empêcher, par des mesures adéquates, toute contamination. Ceci est particulièrement le cas dans l'industrie agro-alimentaire, où des conditions de stérilité doivent être respectées. C'est également important en pathologie humaine, pour effectuer un suivi correct des malades traités à la suite d'une infection à *C. jejuni*, afin d'éviter toute nouvelle rechute.

Enfin, dans le cas d'infections déclarées, il est très important de bien identifier le germe responsable, et ceci rapidement après le déclenchement de la maladie, afin de pouvoir appliquer un traitement correct et efficace qui empêcherait l'évolution de l'infection, voire la propagation d'épidémies. Or, l'identification des *Campylobacter* et la détermination de l'espèce incriminée n'est pas aisée. En effet, leur isolement nécessite des milieux spéciaux et leur détection classique ne se fait actuellement qu'après un enrichissement par culture d'au moins 48 heures. Ceci est très long lorsqu'un diagnostic rapide est nécessaire. D'autre part, étant donné que le diagnostic microbiologique se fait actuellement par des techniques bactériologiques et/ou biochimiques qui exploitent des différences phénotypiques existant entre les différentes espèces, des erreurs de diagnostic peuvent se produire, surtout lorsque des mutants apparaissent pour un caractère donné. Dans le cas précis de *C. jejuni* et *C. coli*, l'unique critère de différenciation est l'hydrolyse de l'hippurate (*C. jejuni* peut l'hydrolyser alors que *C. coli* ne le peut pas), et il arrive parfois que cette distinction ne puisse pas se faire, car il existe des souches *C. jejuni* hippuratenégatives (Hébert et al., J. Clin. Microbiol., 1984, 20, 138-140, Totten et al., J. Clin. Microbiol., 1987, 25, 1747-1752).

Des approches utilisant l'hybridation moléculaire pour identifier l'espèce *C. jejuni* ont été proposées. Cependant, ces méthodes ne permettent l'identification qu'après culture, elles ne sont pas suffisamment sensibles pour détecter cette bactérie dans les prélèvements biologiques. Ainsi, desméthodesd'identification et de classification des *Campylobacter* ont été proposées, utilisant soit des sondes radioactives (Ng et al., Mol. Cell. Probes, 1987, 1, 233-243), soit des sondes non radioactives (Chevrier et ai., J. Clin. Microbiol., 1989, 27, 321-326), mais ces méthodes utilisent des sondes génomiques totales et nécessitent un enrichissement par culture du pathogène à détecter, car le seuil de détection est assez élevé, environ 10⁵ bactéries (Chevrier et al., ci-dessus).

Des recherches de sondes nucléiques spécifiques de *C. jejuni*, dans un but de diagnostic d'espèce, ont été faites par Picken et al. (Mol. Cell. Probes, 1987, 1, 245-259), Korolik et al. (J. Gen. Microbiol., 1988, 134, 521-529) et Zhou et Wang (Zbl. Bakt., 1989, 272, 186-190), mais il subsiste des problèmes de spécificité et les séquences de ces sondes potentielles n'ont pas été déterminées. De même, une autre sonde "spécifique" de *C. jejuni*, constituée d'un oligomère couplé à la phosphatase alcaline et dont la séquence n'a pas été publiée, a été décrite (Jablonski et al., N.A.R., 1986, 14, n° 15), mais sa spécificité n'a été testée que vis-à-vis d'un fragment de *C. jejuni* et non pas contre le génome entier de *C. jejuni* et des autres espèces du genre Campylobacter.

Récemment, une approche pour identifier *C. jejuni* par PCR a été décrite, utilisant des oligonucléotides choisis dans le gène *fla* A de *C. coli* VC167 (Oyofo et al., J. Clin. Microbiol., 1992 30, n° 10, 2613-2619) ; cependant, cette méthode ne permet pas de distinguer *C. jejuni* de *C. coli*.

Les inventeurs ont à présent isolé une séquence nucléique utilisable pour la détection spécifique de l'espèce *Campylobacter jejuni*.

La présente invention a ainsi pour objet une séquence nucléotidique qui hybride spécifiquement avec une séquence d'acide nucléique génomique de *Campylobacter jejuni*, caractérisée en ce qu'elle est choisie parmi la séquence nucléotidique SEQ ID n° 1, la séquence complémentaire de celle-ci, ainsi que les séquences qui en diffèrent par mutation, insertion, délétion ou substitution d'une ou plusieurs bases.

L'invention a également pour objet une séquence nucléotidique comportant tout ou partie de la séquence nucléotidique telle que définie ci-dessus, notamment une séquence nucléotidique choisie parmi la séquence nucléotidique SEQ ID n° 2, et la séquence complémentaire de celle-ci, ainsi que les séquences qui en différent par mutation, insertion, délétion ou substitution d'une ou plusieurs bases.

Par "séquences qui en diffèrent par mutation, insertion, délétion ou substitution d'une ou plusieurs bases", on entend les séquences qui hybrident avec la séquence SEQ ID n° 1, SEQ ID n° 2 ou leurs séquences complémentaires dans les conditions de stringence habituelles définies par SAMBROOK J., FRITSCH E.F. et MANIATIS T. (1989) : Molecular Cloning : A Laboratory Manual, Ed. Cold. Spring Harbor Laboratory 9.47-9.62).

Ces conditions sont déterminées à partir de la température de stringence moyenne Tm.

De préférence, les séquences les plus avantageuses sont celles qui hybrident dans l'intervalle de température (Tm - 15°C) à (Tm - 20°C).

Les séquences an question comportent avantagausement au moins 12 nucléotides.

L'invention a également pour objet les produits d'amplification d'une séquence telle que définie ci-dessus.

L'invention a également pour objet un vecteur de clonage contenant une séquence nucléotidique telle que définie ci-dessus.

Les séquences nucléotidiques définies ci-dessus peuvent être des séquences d'ADN ou des séquences d'ARN.

La taille exacte du fragment de séquence SEQ ID n° 1 est de 147 pb. Cette séquence est spécifique de l'espèce *C. jejuni* et n'hybride pas avec 8 autres espèces représentatives du genre *Campylobacter*.

Le fragment de séquence SEQ ID n° 2 a une longueur de 1189 pb. et hybride très légèrement avec *Campylobacter coli*, mais non avec les 7 autres espèces de *Campylobacter testées*.

Une interrogation des banques de données "Genebank" et "EMBL" n'a fait ressortir aucune homologie entre d'une part les séquences SEQ ID n° 1 et SEQ ID n° 2 et d'autre part les séquences d'ADN connues.

Les séquences SEQ ID n° 1 et SEQ ID n° 2, des parties ou des variants fonctionnellement équivalentes de celles-ci, peuvent être utilisés dans des techniques d'hybridation moléculaire pour la détection et l'identification de *Campylobacter jejuni*.

Les variants fonctionnellement équivalents comprennent des séquences dans lesquelles des paires de bases ont été mutées, délétées, insérées ou substituées, sans que les propriétés essentielles à la spécificité de ces fragments soient affectées.

Les séquences nucléotidiques selon l'invention ont des applications diagnostiques et épidémiologiques en médecine humaine ou vétérinaire, notamment comme sondes nucléiques spécifiques de *Campylobacter jejuni* ou comme amorces oligonucléotidiques pour l'amplification d'une séquence spécifique de *Campylobacter jejuni.*

Les sondes selon l'invention comprennent avantageusement au moins 20 nucléotides consécutifs parmi les séquences ou les fragments de séquences mentionnés ci-dessus.

Les sondes sont des sondes d'ADN ou des sondes d'ARN.

Les séquences nucléotidiques décrites dans cette invention peuvent ainsi être utilisées comme sondes pour détecter spécifiquement et de manière directe des souches de *Campylobacter jejuni* dans un échantillon biologique et permettent la détection des bactéries de l'espèce *Campylobacter jejuni*, quel que soit le biotype auquel appartiennent ces bactéries (il existe 4 "biotypes de Lior", appelés I, II, III et IV, qui classent les bactéries de l'espèce *C. jejuni* selon leur capacité à hydrolyser l'hippurate, à produire de l'H₂S et de la DNase I).

Les sondes oligonucléotidiques décrites détectent également la sous-espèce *C. jejuni* subsp. *doylei*.

Les sondes oligonucléotidiques ne détectent pas l'ADN des bactéries appartenant à d'autres genres susceptibles de se trouver dans le même prélèvement biologique de *C. jejuni* : *Bacteroides fragilis*, *Enterococcus faecalis, Enterococcus faecium* et *Streptococcus agalactiae.*

Les séquences non marquées peuvent être utilisées directement comme sondes, cependant les séquences sont généralement marquées par un élément radioactif (³²p, ³⁵S, ³H, ¹²⁵I) ou par une molécule non-radioactive (biotine, acétylaminofluorène, digoxigénine, 5-bromodésoxyuridine) pour obtenir des sondes utilisables pour de nombreuses applications.

Dans ce dernier cas, on pourra utiliser l'une des méthodes de marquage décrites dans FR 2 422 956 et FR 2 518 755. La technique d'hybridation peut être réalisée de manières diverses (Matthews, J.A. et Kricka, L.J., Anal. Biochem. 1988, 169, 1-25). La méthode la plus générale consiste à immobiliser l'acide nucléique extrait des cellules de *Campylobacter jejuni* sur un support (nitrocellulose, nylon, polystyrène,...) et à incuber, dans des conditions bien définies, l'acide nucléique cible immobilisé avec la sonde. Après l'hybridation, l'excès de sonde est éliminé et les molécules hybrides formées sont détectées par la méthode appropriée (mesure de la radioactivité, de la fluorescence ou de l'activité enzymatique liée à la sonde...).

Dans une autre application, les sondes d'acide nucléique décrites ici peuvent être utilisées comme sondes de capture. Dans ce procédé, la sonde est immobilisée sur un support et sert à capturer par hybridation spécifique l'acide nucléique cible extrait de *C. jejuni*. Si nécessaire, le support solide est séparé de l'échantillon et le duplex formé entre la sonde de capture et l'acide nucléique cible est ensuite détecté grâce à une seconde sonde de détection marquée par un élément facilement détectable.

Lorsqu'une quantité suffisante d'acide nucléique de *Campylobacter jejuni* peut être extraite à partir des prélèvements à analyser, les séquences décrites dans le brevet sont utilisables pour détecter et identifier les souches appartenant à *Campylobacter jejuni* directement dans ces prélèvements. Dans le cas contraire, une culture rapide en milieu liquide peut être réalisée avant l'extraction de l'acide nucléique de *Campylobacter jejuni*, ou bien la petite quantité d'acide nucléique de *Campylobacter jejuni* extrait du prélèvement peut être soumise à une technique d'amplification comme par exemple la technique PCR.

Les séquences SEQ ID n° 1 et SEQ ID n° 2 ou les fragments obtenus à partir de ces séquences peuvent également être utilisés pour sélectionner des amorces oligonucléotidiques, notamment pour la technique PCR.

Cette technique nécessite le choix de paires d'oligonucléotides encadrant le fragment qui doit être amplifié (brevet U.S. n° 4 683 202). Ces amorces oligodésoxyribonucléotidiques ou oligoribonucléotidiques ont avantageusement une longueur comprise entre 18 et 30 et de préférence 18 et 22 nucléotides. L'une des deux amorces est complémentaire du brin (+) de la matrice et l'autre amorce est complémentaire du brin (-). Il est important que ces amorces ne possèdent pas de structure secondaire ou de séquence complémentaire l'une de l'autre. D'autre part, la longueur et la séquence de chaque amorce doivent être choisies de manière à ce que les amorces ne s'hybrident pas avec d'autres acides nucléiques provenant de cellules procaryotes ou eucaryotes, en particulier avec les acides nucléiques des *Campylobacter* n'appartenant pas à l'espèce *jejuni* et avec l'ADN ou l'ARN humain pouvant éventuellement contaminer le prélèvement.

Les amplimères sélectionnés comme amorces spécifiques pour l'amplification de séquences nucléiques de souches appartenant à *Campylobacter jejuni* sont choisis par exemple en suivant la méthode décrite par Griffais et Coll. (Nucleic Acids Res. 1991, 19, 3887-3891).

A partir de la séquence SEQ ID n° 2, les inventeurs ont choisi des oligonucléotides pour réaliser un test PCR. Grâce à ces oligonucléotides, ils ont obtenu une amplification spécifique de *Campylobacter jejuni*, aucune amplification n'était visible avec un acide nucléique d'autres *Campylobacter*.

Un couple d' amorces tout particulièrement préféré est représenté par les oligonucléotides VS15 et VS16 issus de la séquence SEQ ID n° 2, de séquences :
- Oligo VS15 :: ^{5'} GAA TGA AAT TTT AGA ATG GGG ^{3'}
- Oligo VS16 :: ^{5'} GAT ATG TAT GAT TTT ATC CTGC ^{3'}

Les fragments amplifiés peuvent être identifiés après une électrophorèse en gel d'agarose ou de polyacrylamide, ou après une électrophorèse capillaire, ou encore après une technique chromatographique (filtration sur gel, chromatographie hydrophobe ou sur échangeur d'ions). La spécificité de l'amplification peut être contrôlée par hybridation moléculaire en utilisant comme sondes les séquences nucléotidiques SEQ ID n° 1 ou SEQ ID n° 2, des fragments de celles-ci, des plasmides contenant ces séquences ou des fragments de celles-ci, des oligonucléotides complémentaires de ces séquences ou fragments de séquences ou des produits d'amplification. Ces sondes peuvent être marquées ou non par des éléments radioactifs ou par des molécules non radioactives.

La présente invention a également pour objet une méthode de détection de la présence des souches de *Campylobacter jejuni* dans un échantillon biologique, caractérisée par les étapes suivantes :
i) mise on contact de l'échantillon biologique avec un couple de fragments oligonucléotidiques dits amorces, tels que définis précédemment, l'acide nucléique contenu dans l'échantillon ayant, le cas échéant, été préalablement rendu accessible à l'hybridation et dans des conditions permettant une hybridation des amorces à l'acide nucléique des souches appartenant à *Campylobacter jejuni* ;
ii) amplification de l'acide nucléique des souches de *Campylobacter jejuni* ;
iii) mise on évidence de l'amplification de fragments d'acide nucléique correspondant au fragment encadré par les amorces ;
iv) vérification éventuelle de la séquence du fragment amplifié, par exemple par hybridation de sonde spécifique, par séquençage ou par analyse de site de restriction.

La limite de détection, on gel d'aragose après amplification par PCR, est d'une bactérie lorsque des dilutions successives de raison 10 d'une suspension bactérienne de *C. jejuni* sont soumises à l'amplification.

La présente invention a on outre pour objet un kit ou coffret pour la détection de la présence de souches appartenant à *Campylobacter jejuni* dans un échantillon biologique, caractérisé on ce qu'il comporte les éléments suivants :
- un couple de fragments oligonucléotidiques tels que définis précédemment ;
- les réactifs nécessaires pour effectuer une amplification d'acide nucléique de souches appartenant à *Campylobacter jejuni* ;
- éventuellement, un composant permettant de vérifier la séquence du fragment amplifié, plus particulièrement une sonde nucléique telle que définie précédemment.

Ce kit contient plus avantageusement la ou les sondes marquées ou non. Celles-ci peuvent être en solution ou immobilisées sur un support. Le kit peut également contenir les réactifs nécessaires pour la lyse des bactéries et l'extraction des acides nucléiques cibles, ainsi que les solutions d'hybridation et de lavage correspondant à la méthode choisie.

L'invention a également pour objet l'utilisation d'une sonde nucléique telle que définie ci-dessus comme outil épidémiologique, en épidémiologie moléculaire.

En effet, si le fragment spécifique est présent plusieurs fois sur le génome de *C. jejuni*, sa répétitivité peut servir d'outil pour repérer et classer des souches identiques et, évidemment, pour établir des Liens quant à la source et à la propagation de l'infection.

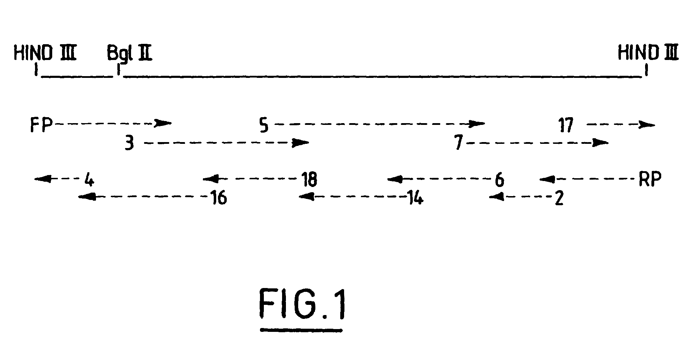

L'invention est illustrée plus en détail dans les exemples qui suivent et à la figure annexée, représentant la stratégie de séquençage de la séquence SEQ ID n° 2 (fragment VS 1) :

### EXEMPLE 1 :

### Construction de la banque génomique de C. jejumi. Criblage de la banque et détermination de la séquence du fragment spécifique :

L'ADN génomique de *C. jejuni* CIP (Collection de l'Institut Pasteur) 70.2 est digéré partiellement par l'endonucléase de restriction *Hind* III en faisant agir 0,06U d'enzyme par µg d'ADN dans le tampon recommandé par le fournisseur pendant 1 heure à 37°C. L'ADN génomique ainsi digéré est séparé par électrophorèse sur gel d'agarose à 0,5%. Les fragments dont la longueur est comprise entre 30 et 40 kb sont électroélués et précipités en éthanol après extractions au phénol/chloroforme (1/1).

Le vecteur est le cosmide réf. pHC79 (fourni par Boehringer). Il est digéré de la même manière et déphosphorylé pour éviter toute autoligation.

La ligation s'effectue en mélangeant 700 ng de vecteur et 1,5 µg de fragments d'ADN de 30/40 kb, le mélange est laissé à 14°C pendant 18 heures après que l'on ait ajouté 1 unité d'ADN ligase de T4 dans un tampon approprié.

Les cosmides recombinants sont encapsidés in vitro et utilisés pour transformer les bactéries (*E. coli* HB 101). Les bactéries transformées sont incubées pendant 1 heure a 37°C en milieu LB, puis étalées sur milieu sélectif Agar contenant 25 µg/ml d'ampillicine. Les colonies résistantes à l'ampicilline sont toutes testées pour leur sensibilité à la tétracycline (le fragment d'ADN de 30/40 kb est inséré dans le vecteur de manière à inactiver le gène de résistance à la tétracycline (Tet) et à conserver le gène de résistance à l'ampicilline (Amp)).

Il est effectué une mini préparation d'ADN des 60 premières colonies transformantes résistantes à l'ampicilline (Amp^{r}) et sensibles à la tétracycline (Tet^{s}) selon la technique de la lyse alcaline. L'ADN de ces préparations est ensuite digéré par l'endonucléase de restriction *Hind* III, analysé en électrophorèse sur gel d'agarose à 0,8%, puis transféré sur des filtres de nylon. L'ADN est fixé de façon irréversible par 3 minutes d'exposition aux UV à 254 nm.

Ces différents filtres sont incubés pendant 16-18 heures à 65°C dans un tampon 6X SSC (1X SSC correspond à 0,15M NaCl et 0,015M citrate de Na) contenant 10% de sulfate de dextrane, une solution de Denhardt 5X concentrée (une solution de Denhardt 1X correspond à 0,02% de Ficoll, 0,02% de polyvinylpyrrolidone et 0,02% d'albumine sérique bovine), 10mM EDTA, 0,5% de SDS, 100 µg/ml d'ADN dénaturé de sperme de saumon et l'ADN génomique, radiomarqué au ³²P par "multipriming" (amorçage multiple), de l'une des trois espèces suivantes : *C. jejuni* CIP 70.2, *C. coli* CIP 70.80 et *C. fetus subsp. fetus* CIP 5396.

Après hybridation, les filtres sont lavés par exemple pendant 2 fois 10 minutes en 2X SSC à 65°C, 1 fois pendant 30 minutes en 2X SSC + 0,1% SDS à 65°C, et enfin 1 fois pendant 10 minutes on 0,1X SSC à 65°C. Les filtres encore humides sont mis on autoradiographie à - 80°C avec écran intensifiant de 15 minutes à 3 jours.

Les résultats de ces hybridations ont permis d'isoler un clone cosmidique contenant un fragment d'environ 1,2 kb nommé VS1. Ce fragment a été cloné dans un vecteur pUC18 (commercialisé par Boehringer) et préparé on gronde quantité. Le plasmide résultant a été dénommé pVS20.

La spécificité du fragment a été vérifiée comme décrit dans l'exemple n° 2.

Le fragment VS1 a été cloné dans le phage M13mp18 et séquencé selon la méthode de Songer on utilisant le kit de séquençage "Séquénase 2.0" (United States Biochemia Corporation). Certaines parties du fragment VS1 ont été séquencées directement sur le plasmide pVS20, après dénaturation alcaline des deux brins d'ADN. Toutes les réactions de séquençage ont été réalisées avec du dATP marque au ³⁵S.

Le schéma de la figure annexée représente les stratégies suivies pour le séquençage du fragment VS1, 2, 3, 4, 5, 6, 7, 14, 16, 17, 18 représentant les différentes amorces utilisées pour le séquençage, FP et RP étant des amorces universelles complémentaires de l'ADN de pUC18 et de M13mp18.

La séquence entière du fragment est la séquence SEQ ID n° 2. La comparaison entre les banques de données "Genebank" et "EMBL" et les 1189 nucléotides du fragment VS1 ainsi déterminés, ne fait ressortir aucune homologie significative avec les séquences connues aujourd'hui.

### EXEMPLE 2 :

### Analyse d'ADN par la technique de Southern, en utilisant comme sondes les séquences d'acide nucléique de l'invention :

La liste et les références des bactéries utilisées dans cette étude sont les suivantes :

### Campylobacter :

*C. jejuni* CIP 70.2
*C. coli* CIP 70.80
*C. lari* CIP 102722
*C. fetus subsp. fetus* CIP 5395
*C. fetus subsp. venerealis* CIP 6829
*C. hyointestinalis* C120
C. curvus (Hôpital des Enfants, Bordeaux)
*C. sputorum subsp. sputorum* CCUG 9728
*C. sputorum subsp. bubulus* CIP 53103
*C. concisus* 18688
*C. fecalis* CIP 12014

### Non-Campylobacter :

*Escherichia coli* HB101
*Helicobacter pylori* CIP 101260
*Salmonella typhimurium* CJ 53
A. *cryaerophilus* CCUG 17801.

### ADN de bactéries n'appartenant pas au genre Campylobacter :

L'ADN de ces bactéries et de *C. jejuni* utilisé comme contrôle positif a été hydrolysé par l'enzyme de restriction *Hind* III, puis les fragments ont été séparés par électrophorèse en gel d'agarose et transférés sur une membrane de nylon Hybond-N. Ces différents fragments d'ADN sont analysés par hybridation moléculaire, en utilisant comme sonde le fragment VS1 marqué au ³²P selon la technique du kit "Random primed DNA Labelling" (Boehringer). L'autoradiographie montre que la seule espèce détectée est *C. jejuni*. Aucune hybridation n'est détectable sur les ADN des espèces non-campylobactériennes, même après 72 heures d'exposition.

### ADN de bactéries appartenant au genre Campylobacter, autres que C. jejuni :

Après culture sur milieu approprié (gélose au sang de mouton à 5%, Biomérieux), les *Campylobacter* sont traités de la manière suivante :

Les bactéries de chaque boite de Petri sont récoltées avec 2 ml de tampon TE-glucose (Tris-HCl pH 8 25 mM, EDTA 10 mM, glucose 50 mM), centrifugées pendant 5 minutes à 5000 g, le culot est redispersé et lavé en TE-glucose puis recentrifugé, les bactéries sont resuspendues dans 100 µl de tampon TE (Tris-HCl 10 mM pH 8, EDTA 1 mM) et l'ADN est extrait selon la technique de Pitcher et al. (Lett. Appl. Microbiol., 1989, 8, 151-156). Les ADN ainsi extraits subissent une digestion totale par l'enzyme *Hind* III. Les fragments obtenus sont ensuite séparés par électrophorèse sur gel d'agarose à 0,8% en TAE avant d'être transférés sur membrane de nylon selon la technique de Southern.

Les fragments transférés sont analysés par hybridation moléculaire. Dans cet exemple, les sondes utilisées étaient, séparément, le fragment VS2 (de séquence SEQ ID n° 1) et le fragment VS3, obtenus après hydrolyse du fragment VS1 par l'enzyme *Bgl* II. Ces deux sondes ont été marquées par du ³²P.

La sonde VS2 détecte spécifiquement l'ADN de *C. jejuni* et n'hybride pas sur les ADN génomiques d'autres *Campylobacter.*

La sonde VS3 détecte également l'ADN de *C. jejuni*, mais hybride aussi très légèrement avec un fragment d'ADN situé sur le génome de *C. coli.* Cette hybridation croisée n'est détectable qu'après 16 heures d'exposition, alors que l'ADN de *C. jejuni* est détectable après seulement 15 minutes d'exposition.

L'ensemble de ces résultats amène à la conclusion que la sonde VS1 (SEQ ID n° 2) détecte spécifiquement l'ADN de *C. jejuni* parmi des ADN de bactéries d'autres genres, et que le fragment VS2 (SEQ ID n° 1) reconnait spécifiquement *C. jejuni* dans le cas d'une identification précise à l'intérieur du genre *Campylobacter*.

### EXEMPLE 3 :

### Amplification enzymatique in vitro de l'ADN de C. jejuni avec les amorces définies à partir de la séquence d'acide nucléique faisant l'objet de l'invention.

### Choix des amorces :

Il a été démontré que c'est essentiellement l'extrémité 3' des amorces oligonucléotidiques qui détermine la spécificité de la PCR (PCR Protocols, M. Innis et col., Academic Press Inc.). Il est donc important que cette région 3' soit parfaitement spécifique de la cible à amplifier.

Etant donné que le génome de *Campylobacter* présente un très faible pourcentage de guanine et cytosine (entre 28 et 38% de G+C), il a été estimé que des amorces dont l'extrémité 3' était riche en G+C pouvaient présenter un important degré de spécificité.

Les inventeurs ont recherché, à l'intérieur de la séquence VS1, des zones riches en G+C et qui sont présentes une seule fois sur VS1. C'est à partir de ces régions que la séquence des amorces a été orientée et complétée de façon à obtenir une longueur d'environ 20 nucléotides.

### Synthèse des amorces oligonucléotidiques :

Les amorces dérivées de la séquence VS1, appelées oligoVS15 et oligoVS16, dont les séquences sont indiquées ci-dessus et ayant une longueur de 21 et 22 nucléotides respectivement, ont été synthétisées sur un appareil automatisé "Cyclone Plus" (Millipore) basé sur la chimie des phosphoramidites. Après la synthèse, la solution d'oligonucléotide est transférée dans un tube et incubée avec de l'hydroxyde d'ammonium concentré pendant 16 heures à 55°C. L'oligonucléotide est précipité à l'éthanol, puis le culot est lavé avec de l'éthanol à 70% et séché. Finalement, le culot est repris dans 1 ml d'eau distillée stérile. La concentration de chaque amorce est déterminée au spectrophotomètre.

### Amplification :

La technique d'amplification, par exemple l'amplification enzymatique *in vitro* (PCR), est réalisée selon le protocole décrit par Saiki et al. (Science, 1988, 239, 487-491) en utilisant 1 µM des oligonucléotides oligoVS15 et oligoVS16 et 30-100 ng d'ADN de différentes souches de *Campylobacter* avec 0,5 unité de Taq polymérase dans un tampon contenant 25 mM de KCl, 20mM de Tris-HCl pH 8,5, 1,5 mM de MgCl₂, 200 µM de désoxyribonucléotides triphosphates et 100 µg/ml d'albumine sérique bovine, le volume final de la réaction étant de 50 µl. Les paramètres des étapes de la PCR ont été choisis de la façon suivante : 5 minutes à 94°C, 1 minute à 60°C, 1 minute à 72°C, puis (1 minute à 94°C, 1 minute à 60°C, 1 minute à 72°C) 28 fois et un dernier cycle 1 minute à 94°C, 1 minute à 60°C, 5 minutes à 72°C. Trente cycles sont ainsi réalisés en utilisant un appareil automatique. Après le dernier cycle, les échantillons sont maintenus à 4°C Jusqu'à l'analyse.

### Analyse électrophorétique sur un gel d'agarose des échantillons amplifiés :

Dix µl des échantillons amplifiés sont déposés sur un gel d'agarose à 2% dans un tampon TBE (0,04 M Tris-borate, 0,001M EDTA) contenant 1 µg/ml de bromure d'éthidium. Les fragments amplifiés sont visualisés sous UV et les gels sont photographiés en utilisant un film Polaroid 667.

On a comparé les résultats obtenus avec, d'une part, différents ADN de *Campylobacter*, d'autre part différents ADN de bactéries n'appartenant pas au genre *Campylobacter*, et les amorces oligoVS15 et oligoVS16. La longueur théorique attendue du fragment amplifié avec ce couple d'amorces est de 358 paires de bases.

Seul l'ADN extrait de *C. jejuni* permet d'obtenir un tel fragment.

Aucun fragment amplifié de la taille attendue n'est visible lorsque l'ADN analysé est extrait des souches suivantes : *C. coli. C. lari, C. fetus subsp. fetus, C. fetus subsp. venerealis. C. hyointestinalis, A. cryaerophilus, C. sputorum subsp. sputorum, C. sputorum subsp. bubulus*, *C. concisus, C. fecalis*, *E. coli*, *H. pylori*, *S. typhimurium*, *C. curvus*, ou de cellules humaines.

Dans le cas de *C. fetus subsp. fetus*, un fragment de haut poids moléculaire a été amplifié, mais de façon non-spécifique, car après transfert sur membrane de nylon, ce fragment n'hybride pas avec la sonde VS1 marquée au ³²P.

D'autre part, la technique d'identification de *C. jejuni* par PCR décrite ci-dessus a été utilisée pour tester 15 souches de *C. jejuni* isolées sur des pintades. L'amplification a été réalisée après subculture des souches et extraction de l'ADN. Toutes les souches ont été identifiées comme étant des *C. jejuni*, ce qui est en accord avec les tests biochimiques effectués au préalable.

### Détermination de la sensibilité du test PCR

Afin de déterminer le seuil de détection absolu de l'ADN de *C. jejuni* par PCR avec le couple d'oligonucléotides décrit ici, on a soumis à l'amplification par PCR plusieurs dilutions successives de raison 10 d'une suspension bactérienne de *C. jejuni*. L'amplification comprend 40 cycles, les cycles étant réalisés dans les conditions décrites ci-dessus et est effectuée après mélange des dilutions à un tampon de lyse approprié suivi d'un traitement thermique (incubation pendant 15 minutes à 65°C, puis pendant 10 minutes à 95°C). La composition du tampon de lyse est la suivante : Tris-HCl 10mM, EDTA 1mM, pH8 contenant 0,5% Tween 20, 0,5% Nonidet-P40 et 500 µg/ml protéinase K.

Un volume de 100 µl de chaque dilution, identique à celui soumis à la lyse, a été étalé sur boîte de Petri contenant un milieu de culture pour *Campylobacter*, et une numération des colonies a été effectuée après 48 heures d'incubation. Dans ces conditions, la limite de détection a été de sept bactéries.

Avec la méthode PCR, en soumettant à l'amplification 10 µl de chaque dilution, la limite de détection est statistiquement d'une bactérie (Dilutions théoriques de 3,5 x 10⁷ bactéries / 100 µl à 3,5 bactéries / 100 µl respectivement, correspondant à des numérations sur boite de 2,5 x 10⁷, 10⁶, 10⁵, 1,5 x 10⁴, 560, 32, 7 et 0 bactéries dans 100 µl respectivement).

En conclusion, il est important de noter que ce couple d'amorces permet de détecter spécifiquement l'espèce *C. jejuni*, ce qui laisse penser que des isolats de malades et des échantillons biologiques infectés par *C. jejuni* peuvent être identifiés, rendant ainsi la méthode utilisable dans le domaine de la bactériologie clinique et en médecine vétérinaire.

La méthode de détection de *C. jejuni* selon l'invention peut également être utilisée pour mettre en évidence la présence de *C. jejuni* dans des aliments (escalope de poulet, viande de boeuf, lait, eau) à l'aide des sondes oligonucléotidiques décrites précédemment.

Dans ce cas, l'élimination des débris organiques alimentaires grossiers par centrifugation à basse vitesse est nécessaire avant la lyse des bactéries, afin d'améliorer les résultats du test PCR et d'éviter d'éventuels faux négatifs dus à une inhibition de la réaction d'amplification.

LISTE DES SEQUENCES

I - INFORMATIONS GENERALES

(1) DEMANDEUR : Institut Pasteur

(2) TITRE DE L'INVENTION :

Séquences nucléotidiques hybridant spécifi-
quement avec une séquence nucléique génomique
de Campylobacter jejuni.

(3) NOMBRE DE SEQUENCES : 4

II - INFORMATIONS POUR SEQ. ID N° 1 :
CARACTERISTIQUES DE LA SEQUENCE :
TYPE : Nucléotide
LONGUEUR : 147 paires de bases
NOMBRE DE BRINS : simple
CONFIGURATION : linéaire
TYPE DE MOLECULE : ADN
ORGANISME : Campylobacter jejuni
NOM : VS2
DESCRIPTION DE LA SEQUENCE :

10 20 30 40 50
AAGCTTGTGA TACTTTTAAG TGCTATAGAA AGTGAAAATG AAATTTCTTT

60 70 80 90 100
AGCAGGCATA TATAGAGCGT ATTGTTCCAA ATTTGATTTA AAGAATGAAA

110 120 130 140
TTTTAGAATG GGGTCTTAAA ATATTTAAAA ACAATAATGC CTTAAAA

III - INFORMATIONS POUR SEQ. ID N° 2 :
CARACTERISTIQUES DE LA SEQUENCE :
TYPE : Nucléotide
LONGUEUR : 1189 paires de bases
NOMBRE DE BRINS : simple
CONFIGURATION : linéaire
TYPE DE MOLECULE : ADN
ORGANISME : Campylobacter jejuni
NOM : VS1
DESCRIPTION DE LA SEQUENCE

10 20 30 40 50
AAGCTTGTGA TACTTTTAAG TGCTATAGAA AGTGAAAATG AAATTTCTTT

60 70 80 90 100
AGCAGGCATA TATAGAGCGT ATTGTTCCAA ATTTGATTTA AAGAATGAAA
********

110 120 130 140 150
TTTTAGAATG GGGTCTTAAA ATATTTAAAA ACAATAATGC CTTAAAAGAT
**************

160 170 180 190 200
CTTGTAGAAA AAGAAGATAT ATACAATCCT ATTGTTGTAA GTAGTTTGGT

210 220 230 240 250
TTCTAAGCTA GAAAATTTAG AAAATTTAGA GCTTTTATAT ACTTTAACTT

260 270 280 290 300
GGCTAAAGGC TAAGGCTTTA AATTATAATG CTTTTTATTT TAGAGTTCTT

310 320 330 340 350
GATAAACTTT TAGAAAATGC AAAACAAGGT TTTGAAGATG AAAATCTACT

360 370 380 390 400
TGAAGAAAGT GCAAGAAGGG TAAAAAAAGA ATTAACACTT AAAAGAAGTA

410 420 430 440 450
AGATTTTTTT AGAGCAAGAT GAAATTTTGC AGGATAAAAT CATACATATC
************************

460 470 480 490 500
AAATCAAATC TTTTTATTAT AAAAAATACT TTTGAAGATA TTGTTATGAT

510 520 530 540 550
TTCTAAATTA GCCAAAGAAA ATGATTTTAA ATTTTGGTTT AGTAATGAAA

560 570 580 590 600
CAAATCTTAG TTTGCAAATT GTTGCACCAC TTCATTTTAA TATTGCCATT

610 620 630 640 650
ATTTTAAGTT CTTTAACAAA TTTAAATCTT ATTTTTATGA ATTTTTTTGA

660 670 680 690 700
ACTTTTTGAT GATAAAATTT ATTTAAGGTT TGAATATGAT AATATTATCA

710 720 730 740 750
GTGATGAGCA AAAACTAAAA CTTTGTGAGC TTTTAAATTC AAATCTTTCT

760 770 780 790 800
GGTTTTAATT TGAAAAAAAT TAAAAAGCCA ATCATTAAAA AAGAGGAGTT

810 820 830 840 850
AAAATTAGAC TTAAACTATT CTAAAATGTA TGCCAAATTA GGTCTTAATA

860 870 880 890 900
CTAAAGATCA GCAAGGTTTA ATGGCGTATT TGATGAATGT TTTTAATGAA

910 920 930 940 950
CTTGAACTTG TTTTATGTGC AGCAAAAATT CAAACCATAA GACAAAGGAC

960 970 980 990 1000
GCGTAATATT TTTATTTTTC AAAAGAATGA AAAATTAGAA CATAGCGAGC

1010 1020 1030 1040 1050
AAAAGTTAGT TAATTTATTA ATAAGTGAGT AAAAAAATGT GTGGAATCGT

1060 1070 1080 1090 1100
AGGCTATATA GGAAATAATG AAAAAAAACA AATTATACTA AATGGACTTA

1110 1120 1130 1140 1150
AAGAATTAGA ATATCGTGGC TATGATAGTG CGGGTATGGC AGTGATGCAA

1160 1170 1180
GAAGGCGAAC TTAGTTTTTT TAAAGCTGTA GGAAAGCTT

IV - INFORMATIONS POUR SEQ. ID N° 3 :
CARACTERISTIQUES DE LA SEQUENCE :
TYPE : Nucléotide
LONGUEUR : 21 bases
NOMBRE DE BRINS : simple
CONFIGURATION : linéaire
TYPE DE MOLECULE : ADN
ORGANISME : Campylobacter jejuni
NOM : OligoVS15
DESCRIPTION DE LA SEQUENCE :
5' GAA TGA AAT TTT AGA ATG GGG 3'

V - INFORMATIONS POUR SEQ. ID N° 4 :
CARACTERISTIQUES DE LA SEQUENCE :
TYPE : Nucléotide
LONGUEUR : 22 bases
NOMBRE DE BRINS : simple
CONFIGURATION : linéaire
TYPE DE MOLECULE : ADN
ORGANISME : Campylobacter jejuni
NOM : OligoVS16
DESCRIPTION DE LA SEQUENCE :
5' GAT ATG TAT GAT TTT ATC CTGC 3'

## Revendications

1. Séquences nucléotidiques qui hybrident spécifiquement avec une séquence d'acide nucléique génomique de *Campylobacter jejuni*, caractérisée en ce qu'elle est choisie parmi la séquence nucléotidique SEQ ID n° 1, la séquence complémentaire de celle-ci, ainsi que les séquences qui hybrident avec la séquence SEQ ID n° 1 ou sa séquence complémentaire.

2. Séquence nucléotidique comportant tout ou partie de la séquence nucléotidique selon la revendication 1.

3. Séquence nucléotidique comportant une séquence nucléotidique selon la revendication 1, caractérisée en ce qu'elle est choisie parmi la séquence nucléotidique SEQ ID n° 2, la séquence complémentaire de celle-ci, ainsi que les séquences qui hybrident avec la séquence SEQ ID n° 2 ou sa séquence complémentaire.

4. Produits d'amplification de l'une des séquences selon l'une des revendications 1 à 3.

5. Vecteur de clonage caractérisé en ce qu'il contient une séquence nucléotidique selon l'une des revendications 1 à 3.

6. Sonde nucléique spécifique des acides nucléiques de Campylobacter jejuni, caractérisée en ce qu'elle comprend au moins 20 nucléotides consécutifs choisis parmi les séquences nucléotidiques selon l'une des revendications 1 à 4.

7. Sonde nucléique spécifique des acides nucléiques de *Campylobacter jejuni* selon la revendication 6, caractérisée en ce qu'elle est choisie parmi la séquence nucléotidique SEQ ID n° 1, la séquence nucléotidique SEQ ID n° 2, les séquences complémentaires de celles-ci, ainsi que les séquences qui hybrident avec la séquence SEQ ID n° 1 ou SEQ ID n° 2 ou leurs séquences complémentaires.

8. Sonde nucléique selon la revendication 6 ou 7, caractérisée en ce qu'elle est immobilisée sur un support et utilisée comme sonde de capture.

9. Couples d'amorces oligonucléotidiques spécifiques pour l'amplification d'une séquence nucléique de *Campylobacter jejuni*, caractérisés en ce qu'ils comprennent des paires d'oligonucléotides ayant de 18 à 30 nucléotides, de préférence 18 à 22 nucléotides choisis parmi les séquences selon l'une des revendications 1 à 4.

10. Couple d'amorces oligonucléotidiques spécifiques pour l'amplification d'une séquence nucléique de *Campylobacter jejuni* selon la revendication 9, caractérisé en ce qu'il est constitué par les paires oligonucléotidiques de séquences :
^{5'} GAA TGA AAT TTT AGA ATG GGG ^{3'}
^{5'} GAT ATG TAT GAT TTT ATC CTGC ^{3'}

11. Méthode de détection de la présence de *Campylobacter jejuni* dans un échantillon biologique, caractérisée par les étapes suivantes :
a) mise en contact de l'échantillon biologique avec un couple d'amorces selon la revendication 9 ou 10, l'acide nucléique de *Campylobacter jejuni* contenu dans l'échantillon biologique ayant, le cas échéant, été rendu préalablement accessible à une hybridation, et dans des conditions permettant une hybridation des amorces à l'acide nucléique appartenant à *Campylobacter jejuni* ;
b) amplification de l'acide nucléique appartenant à *Campylobacter jejuni* ;
c) mise en évidence de l'amplification de fragments d'acide nucléique de *Campylobacter jejuni* correspondant au fragment encadré par les amorces ;
d) vérification éventuelle de la séquence du fragment amplifié, par exemple par hybridation de sonde spécifique, par séquençage ou par analyse de site de restriction.

12. Méthode de détection de la présence de *Campylobacter jejuni* dans un échantillon d'aliments, caractérisée par les étapes suivantes :
a) centrifugation à basse vitesse pour éliminer les débris organiques alimentaires grossiers,
b) mise en contact de l'échantillon d'aliments avec un couple d'amorces selon la revendication 9 ou 10, l'acide nucléique de *Campylobacter jejuni* contenu dans l'échantillon d'aliments ayant, le cas échéant, été rendu préalablement accessible à une hybridation, et dans des conditions permettant une hybridation des amorces à l'acide nucléique appartenant à *Campylobacter jejuni* ;
c) amplification de l'acide nucléique appartenant à *Campylobacter jejuni* ;
d) mise en évidence de l'amplification de fragments d'acide nucléique de *Campylobacter jejuni* correspondant au fragment encadré par les amorces

13. Kit de détection de la présence de *Campylobacter jejuni* dans un échantillon biologique ou d'un aliment, caractérisé en ce qu'il comprend les éléments suivants :
- un couple d'amorces selon la revendication 9 ou 10 ;
- les réactifs nécessaires pour effectuer une amplification d'un acide nucléique de *Campylobacter jejuni* ;
- éventuellement un composant permettant de vérifier la séquence du fragment amplifié, plus particulièrement une sonde nucléique selon l'une des revendications 6 à 8.

14. Utilisation d'une sonde nucléique selon l'une des revendications 6 à 8 comme outil épidémiologique pour repérer et classer des Souches de *Campylobacter jejuni* à des souches particulières de *Campylobacter jejuni*.

## Claims

1. Nucleotide sequences which hybridise specifically with a sequence of genomic nucleic acid of *Campylobacter jejuni,* characterised by the fact that it is selected from the nucleotide sequence SEQ ID no. 1, the complementary sequence of the latter, and also the sequences which hybridise with the sequence SEQ ID no. 1 or its complementary sequence.

2. Nucleotide sequence comprising all or part of the nucleotide sequence according to claim 1.

3. Nucleotide sequence comprising a nucleotide sequence according to claim 1, characterised by the fact that it is selected from the nucleotide sequence SEQ ID no. 2, the complementary sequence of the latter, and also the sequences which hybridise with the sequence SEQ ID no. 2 or its complementary sequence.

4. Products of amplification of one of the sequences according to any of claims 1 to 3.

5. Cloning vector characterised by the fact that it contains a nucleotide sequence according to any of claims 1 to 3.

6. Specific nucleic probe of the nucleic acids of *Campylobacter jejuni*, characterised by the fact that it includes at least 20 consecutive nucleotides selected from the nucleotide sequences according to any of claims 1 to 4.

7. Specific nucleic probe of the nucleic acids of *Campylobacter jejuni* according to claim 6, characterised by the fact that it is selected from the nucleotide sequence SEQ ID no. 1, nucleotide sequence SEQ ID no. 2, the complementary sequences of these latter, and also the sequences which hybridise with the sequence SEQ ID no. 1 or SEQ ID no. 2 or their complementary sequences.

8. Nucleic probe according to claim 6 or 7, characterised by the fact that it is immobilised on a support and used as a capture probe.

9. Specific oligonucleotide base pairs for the amplification of a nucleic sequence of *Campylobacter jejuni*, characterised by the fact that they include pairs of oligonucleotides having from 18 to 30 nucleotides, preferably 18 to 22 nucleotides selected from the sequences according to any of claims 1 to 4.

10. Specific oligonucleotide base pairs for the amplification of a nucleic sequence of *Campylobacter jejuni*, according to claim 9, characterised by the fact that it consists of pairs of oligonucleotides of sequences:
^{5'}GAA TGA AAT TTT AGA ATG GGG^{3'}
^{5'}GAT ATG TAT GAT TTT ATC CTGC^{3'}

11. Method for detecting the presence of *Campylobacter jejuni* in a biological sample, characterised by the following stages:
a) placing the biological sample in contact with a base pair according to claim 9 or 10, the nucleic acid of *Campylobacter jejuni* contained in the biological sample having, if necessary, been previously rendered accessible to a hybridisation, and in conditions which allow a hybridisation of the nucleic acid bases belonging to the *Campylobacter jejuni*;
b) amplification of the nucleic acid belonging to the *Campylobacter jejuni*;
c) highlighting the amplification of the fragments of nucleic acid of *Campylobacter jejuni* corresponding to the fragment bracketed by the bases;
d) any verification of the sequence of the amplified fragment, for example by hybridisation of specific probe, by sequencing or by analysis of the restriction site.

12. Method for detecting the presence of *Campylobacter jejuni* in a sample of food, characterised by the following stages:
a) low-speed centrifuging to eliminate coarse organic food debris,
b) placing in contact of the food sample with a base pair according to claim 9 or 10, the nucleic acid of *Campylobacter jejuni* contained in the food sample having, if necessary, been previously rendered accessible to a hybridisation, and in conditions which allow a hybridisation of the nucleic acid bases belonging to the *Campylobacter jejuni*;
c) amplification of the nucleic acid belonging to the *Campylobacter jejuni*;
d) highlighting the amplification of the fragments of nucleic acid of *Campylobacter jejuni* corresponding to the fragment bracketed by the bases;

13. Kit for the detection of the presence of *Campylobacter jejuni* in a biological sample or a foodstuff, characterised by the fact that it includes the following elements:
- a base pair according to claim 9 or 10;
- the necessary reagents to effect an amplification of a nucleic acid of *Campylobacter jejuni*;
- if necessary, a component allowing verification of the sequence of the amplified fragment, more particularly a nucleic probe according to any of claims 6 to 8.

14. Use of a nucleic probe according to any of claims 6 to 8 as an epidemiological tool to mark and classify strains of *Campylobacter jejuni* as individual strains of *Campylobacter jejuni*.

## Patentansprüche

1. Nukleotidsequenzen, die spezifisch mit einer genomischen Nukleinsäuresequenz von *Campylobacter jejuni* hybridisieren, dadurch gekennzeichnet, daß sie ausgewählt sind aus der Nukleotidsequenz SEQ ID Nr. 1, deren komplementärer Sequenz sowie den Sequenzen, die mit der Sequenz SEQ ID Nr. 1 oder deren komplementärer Sequenz hybridisieren.

2. Nukleotidsequenz, die die Nukleotidsequenz nach Anspruch 1 vollständig oder teilweise umfaßt.

3. Nukleotidsequenz, die eine Nukleotidsequenz nach Anspruch 1 umfaßt, dadurch gekennzeichnet, daß sie ausgewählt ist aus der Nukleotidsequenz SEQ ID Nr. 2, deren komplementärer Sequenz sowie den Sequenzen, die mit der Sequenz SEQ ID Nr. 2 oder deren komplementärer Sequenz hybridisieren.

4. Amplifizierte Produkte von einer der Sequenzen nach einem der Ansprüche 1 bis 3.

5. Klonierungsvektor, dadurch gekennzeichnet, daß er eine Nukleotidsequenz nach einem der Ansprüche 1 bis 3 enthält.

6. Für Nukleinsäuren von *Campylobacter jejuni* spezifische Nukleinsonde, dadurch gekennzeichnet, daß sie mindestens 20 aufeinander folgende Nukleotide enthält, ausgewählt aus den Nukleotidsequenzen gemäß einem der Ansprüche 1 bis 4.

7. Für Nukleinsäuren von *Campylobacter jejuni* spezifische Nukleinsonde nach Anspruch 6, dadurch gekennzeichnet, daß sie ausgewählt ist aus der Nukleotidsequenz SEQ ID Nr. 1, der Nukleotidsequenz SEQ ID Nr. 2, deren komplementären Sequenzen sowie den Sequenzen, die mit der Sequenz SEQ ID Nr. 1 oder SEQ ID Nr. 2 und deren komplementären Sequenzen hybridisieren.

8. Nukleotidsonde nach Anspruch 6 oder 7, dadurch gekennzeichnet, daß sie auf einem Träger immobilisiert ist und als Fängersonde verwendet wird.

9. Oligonukleotid-Köderpaare zur spezifischen Amplifizierung einer Nukleinsequenz von *Campylobacter jejuni*, dadurch gekennzeichnet, daß sie Oligonukleotidpaare mit 18 bis 30 Nukleotiden, vorzugsweise 18 bis 22 Nukleotiden, ausgewählt aus den Sequenzen nach einem der Ansprüche 1 bis 4, umfassen.

10. Oligonukleotid-Köderpaar zur spezifischen Amplifizierung einer Nukleinsequenz von *Campylobacter jejuni* nach Anspruch 9, dadurch gekennzeichnet, daß sie aus den Oligonukleotidpaaren der Sequenzen
^{5'} GAA TGA AAT TTT AGA ATG GGG ^{3'}
^{5'} GAT ATG TAT GAT TTT ATC CTGC ^{3'}
gebildet ist.

11. Verfahren zum Nachweis von *Campylobacter jejuni* in einer biologischen Probe, gekennzeichnet durch die folgenden Stufen:
a) Inkontaktbringen der biologischen Probe mit einem Köderpaar nach Anspruch 9 oder 10, die Nukleinsäure von *Campylobacter jejuni*, die in der biologischen Probe enthalten ist, wurde, sofern vorhanden, zuvor zur Hybridisierung befähigt und in den Zustand gebracht, der eine Hybridisierung der Köder mit der in *Campylobacter jejuni* auftretenden Nukleinsäure erlaubt;
b) Amplifizierung der in *Campylobacter jejuni* auftretenden Nukleinsäure;
c) Nachweis der Amplifizierung der Nukleinsäurefragmente von *Campylobacter jejuni*, die dem durch die Köder eingefügten Fragment entsprechen;
d) gegebenenfalls Überprüfung der amplifizierten Sequenz, beispielsweise durch Hybridisierung mit einer spezifischen Sonde, durch Sequenzierung oder durch Analyse der Schnittstellen.

12. Verfahren zum Nachweis von *Campylobacter jejuni* in einer Lebensmittelprobe, gekennzeichnet durch die folgenden Stufen:
a) Zentrifugieren bei niedriger Geschwindigkeit zur Entfernung der organischen, großen Lebensmittelreste,
b) Inkontaktbringen der Lebensmittelproben mit einem Köderpaar nach Anspruch 9 oder 10, die Nukleinsäure von *Campylobacter jejuni*, die in den Lebensmittelproben enthalten ist, wurde, sofern vorhanden, zuvor zur Hybridisierung befähigt und in den Zustand gebracht, der eine Hybridisierung der Köder mit der in *Campylobacter jejuni* auftretenden Nukleinsäure erlaubt.
c) Amplifizierung der in *Campylobacter jejuni* auftretenden Nukleinsäure;
d) Nachweis der Amplifizierung der Nukleinsäurefragemente von *Campylobacter jejuni*, die dem durch die Köder eingefügten Fragment entsprechen.

13. Kit zum Nachweis von *Campylobacter jejuni* in einer biologischen Probe oder einer Lebensmittelprobe, dadurch gekennzeichnet, daß er die folgenden Bestandteile enthält:
- Ein Köderpaar nach Anspruch 9 oder 10;
- die zur Amplifizierung einer Nukleinsäure von *Campylobacter jejuni* erforderlichen Reagenzien;
- gegebenenfalls einen Bestandteil zur Überprüfung der amplifizierten Sequenz, insbesondere eine Nukleinsonde nach einem der Ansprüche 6 bis 8.

14. Verwendung einer Nukleinsäure nach einem der Ansprüche 6 bis 8 als epidemiologisches Werkzeug zum Markieren und Klassifizieren von Stämmen von *Campylobacter jejuni* als besondere Stämme von *Campylobacter jejuni*.
